Europäisches Patentamt

European Patent Office

Office européen des brevets

(11) Veröffentlichungsnummer: **0 354 345 B1**

(12) **EUROPÄISCHE PATENTSCHRIFT**

(45) Veröffentlichungstag der Patentschrift: **01.12.93**

(21) Anmeldenummer: **89112173.3**

(22) Anmeldetag: **04.07.89**

(51) Int. Cl.⁵: **C09H 9/04**, C08L 89/06, B29C 47/00

(54) **Gelatinegranulat sowie Verfahren und Vorrichtung zu seiner Herstellung.**

(30) Priorität: **10.08.88 DE 3827061**

(43) Veröffentlichungstag der Anmeldung:
**14.02.90 Patentblatt 90/07**

(45) Bekanntmachung des Hinweises auf die Patenterteilung:
**01.12.93 Patentblatt 93/48**

(84) Benannte Vertragsstaaten:
**AT BE CH DE ES FR GB GR IT LI LU NL SE**

(56) Entgegenhaltungen:
**EP-A- 0 092 908**
**GB-A- 875 942**

**SOVIET INVENTIONS ILLUSTRATED, Woche 8435, 10. Oktober 1984, Sektion C, Nr. 84-218319/35, Derwent Publications Ltd, London, GB**

(73) Patentinhaber: **Deutsche Gelatine-Fabriken Stoess AG**
**Postfach 12 53**
**D-69402 Eberbach(DE)**

(72) Erfinder: **Koepff, Peter, Dr.**
**Bergstrasse 142**
**D-6900 Heidelberg(DE)**
Erfinder: **Bräumer, Klaus, Dr.**
**Allensteiner Strasse 6**
**D-6930 Eberbach(DE)**
Erfinder: **Stahl, Helmut**
**Am Wäldchen 1**
**D-6120 Michelstadt(DE)**

(74) Vertreter: **Hoeger, Stellrecht & Partner**
**Uhlandstrasse 14 c**
**D-70182 Stuttgart (DE)**

**Beschreibung**

Die Erfindung betrifft ein Gelatinegranulat sowie Verfahren und Vorrichtungen zu seiner Herstellung.

Gelatine, die gewöhnlich in Gestalt von Gelatinepulver oder als Gelatinehydrolysat vorliegt, muß bekanntlich bei allen Verarbeitungen im technischen Maßstab einem Auflösungsvorgang in Wasser unterzogen werden, wobei relativ viel Wasser benötigt wird, welches dann dem herzustellenden Endprodukt durch schonende Trocknung wieder entzogen werden muß. All dies erfordert einen hohen apparativen und energieintensiven Aufwand. Das Gelatineendprodukt enthält außerdem eingeschlossene Luftbläschen, welche beim Lösevorgang freigesetzt werden. Um die Luftbläschen zu entfernen ist es erforderlich, die Gelatinelösung vor der weiteren Verarbeitung zu entgasen, was einen weiteren apparativen und zeitlichen Aufwand bedingt. Bei der Trocknung des Gelatineproduktes entsteht weiterhin ein hoher Schrumpf, der ebenfalls zu technischen Problemen Anlaß gibt. Eine Herstellung von Formkörpern aus Gelatine oder Gelatinehydrolysat mit einem Molekulargewicht kleiner als 10 kD (Kilodalton) ist unmöglich.

Es wurde auch bereits vorgeschlagen, Gelatine, welche in der zuvor beschriebenen Weise vorbehandelt, also insbesondere mit Wasser verarbeitet war, zu Formprodukten zu extrudieren, spritzzugießen und tiefzuziehen (EP-PS-0 090 600; GB-A-875 942), ohne daß diese Verfahren bisher in größerem Maßstabe industriell genutzt werden konnten. Ein Grund für letzteres dürfte darin bestehen, daß die Gelatine in Wasser gelöst, also vorgequollen werden muß, und die Mischung über längere Zeit hinweg nicht lagerfähig ist. Außerdem kann die vorgequollene Masse im üblichen Schneckenextruder nicht stippenfrei (homogen) aufgeschmolzen werden. Ferner entstehen Probleme bei der Dosierung der Masse in den Extruder, und vor allem muß das vorher zugemischte Wasser aus dem Endprodukt durch Trocknung wieder entfernt werden.

Der Erfindung liegt die Aufgabe zugrunde, den geschilderten Mängeln bei der Herstellung geformter Gelatineerzeugnisse abzuhelfen und insbesondere ein neues Gelatine-Ausgangsmaterial vorzuschlagen, welches eine thermoplastische Verarbeitung in Extrudern und ähnlichen Maschinen, wie sie in der Kunststoffindustrie eingesetzt werden, gestattet.

Die Aufgabe wird erfindungsgemäß durch ein Gelatinegranulat gelöst, welches massive Gelatinekörper umfaßt mit einem Wassergehalt von 1 - 12 Gew%, mit einer Korngröße von 0,1 mm bis 10 mm, mit einem Gehalt an Lufteinschlüssen von weniger als 1 Vol% und mit einem Schmelzindex größer als 1 g/10 min bei 110°C Prüftemperatur, 30 kg Gesamtbelastungsgewicht und unter Verwendung einer Standarddüse gemäß DIN A 53 735, Seite 2 mit einem Verhältnis Länge : Durchmesser von 8 mm : 2 mm.

Die Verarbeitung dieses Gelatinegranulats erfolgt bei demjenigen Endwassergehalt, den die durch Plastifizierung des Granulats hergestellten Formprodukte haben. Eine Vorquellung des Gelatinegranulats in Wasser ist nicht erforderlich. Desgleichen entfällt eine nachträgliche Trocknung. Die Herstellung von Formkörpern, Schäumen, Folien usw. dauert bei Verwendung des erfindungsgemäßen Gelatinegranulats - genauso wie mit konventionellen, thermoplastischen Kunststoffen - nur wenige Sekunden, da die Verarbeitung aus der Schmelze und nicht aus der Lösung erfolgt. Das erfindungsgemäße Gelatinegranulat verhält sich also wie ein Thermoplast.

Die Schrumpfung der mit dem Gelatinegranulat hergestellten Formkörper ist sehr gering und über Additive, beispielsweise Weichmacher, einstellbar.

Die nachstehende Beschreibung bevorzugter Ausführungsformen der Erfindung dient im Zusammenhang mit den nachstehend angegebenen Beispielen der weiteren Erläuterung.

Erfindungsgemäß wird thermoplastisches Gelatinegranulat wie folgt hergestellt: man geht aus von handelsüblicher Pulvergelatine und/oder handelsüblichem pulverförmigem Gelatinehydrolysat, wobei der Wassergehalt in beiden Fällen zwischen 1 und 12 Gew%, vorzugsweise zwischen 8 und 12 Gew%, liegen kann. Die Ausgangssubstanz wird ohne Vorkonditionierung in einen Extruder, vorzugsweise einen gleichläufigen Zweischneckenextruder mit einem Längen/Durchmesser (L/D) -Verhältnis von mindestens 35 eingegeben. Zusammen mit der Ausgangssubstanz oder an besonderen Dosierstellen können, falls erforderlich, 0,1 bis 80 Gew% Additive zugesetzt werden, insbesondere Weichmacher (1 - 50 Gew%), Vernetzungsmittel oder Härter (0,1 - 10 Gew%), Füllstoffe (0 - 80 Gew%) und/oder Farbstoffe (0,001 - 5 Gew%).

An Weichmachern kommen beispielsweise in Frage: Glycerin, Mannit, Sorbit, Fett, Fettsäure, Seife, modifizierte oder native Stärke, Äthylenglykol, Polyäthylenglykol, Mono-, Di-, Triglycerinacetat, Zuckerfettsäureester, Dimethylensulfoxid, 2,2,2-Trifluoräthanol und/oder Propylenglykol.

Die Menge an Weichmacher kann insbesondere auch 5 - 30 Gew%, vorzugsweise 5 - 10 Gew% betragen.

An Vernetzungsmittel (Härter), dessen Zusatzmenge auch im Bereich von 0,5 - 5 Gew% liegen kann, kommen in Frage: Aldehyd, Dialdehyd, Moleküle mit mehrfacher Aldehydfunktion, Diisocyanat, Dialdehydstärke, Acrylsäure, Acrylat, Hexamethylendiamin, Styrol, reaktives Melaminderivat und/oder Acrolein.

Die zugesetzte Menge an Füllstoffen kann auch 20 - 60 Gew%, vorzugsweise 30 - 50 Gew% betragen. In Frage kommen in erster Linie: Calciumcarbonat, Zellmehl (gemahlenes Holzmehl, Baumwolle oder dergleichen), Kohlehydrat (Stärke und/oder Zucker), Dicalciumphosphat, feinstgemahlenes Fleisch- oder Knochenmehl, Fischmehl, Milcheiweiß, Sojamehl, Fett, Fettsäure, Polyvinylalkohol, Talkum, Polyäthylen, Polypropylen, Polyamid und/oder Polyester.

Die Menge an zugesetztem Farbstoff kann auch zwischen 0,1 - 3 Gew%, vorzugsweise zwischen 0,5 und 2 Gew% liegen.

Die Ausgangssubstanz mit oder ohne Additive wird im Extruder während einer Zeitdauer von 5 Sekunden bis 30 Minuten bei einer Temperatur zwischen 30 und 200° C plastifiziert. Je nach Beschaffenheit der Ausgangsmischung können auch Plastifizierzeiten zwischen 0,5 und 10 min oder zwischen 1 und 5 min ausreichend sein. Geeignete Arbeitstemperaturen liegen auch in den Bereichen zwischen 5 und 150° C oder 70 bis 130° C.

Während der Plastifizierung wird auf die Masse ein Druck zwischen 1 und 300 bar, vorzugsweise 3 bis 100 bar oder 5 bis 50 bar ausgeübt.

Über eine oder mehrere am Extruder vorgesehene Düsen, vorzugsweise Rundlochdüsen, werden endlose Stränge extrudiert, die nach Abkühlung im Luftstrom mit Schneidmühlen oder anderen Stranggranulatoren (wie bei Kunststoff üblich) mit gleicher oder unterschiedlicher Korngröße granuliert werden. Die Korngröße kann zwischen 0,1 und 10 mm liegen. Die untere Grenze der Korngröße beträgt vorzugsweise 1 oder 2 mm.

Das Überraschende bei diesem Herstellungsverfahren ist, daß beispielsweise Pulvergelatine ohne Vorkonditionierung im Extruder verarbeitet werden kann, und daß Extrudate mit hohem Additivgehalt stippenfrei erzeugt werden können.

Aufgrund der Plastifizierung des Ausgangsmaterials unter Druck und unter Anwendung von Scherkräften im Schneckenextruder sowie der Stabilisierung und Formgebung bei Temperaturen oberhalb des Glasübergangspunktes bzw. des Schmelzpunktes der Ausgangsgelatine bleiben die gesamten Proteinmoleküle der Gelatine nach der Abkühlung in einem Zustand geringerer Ordnung, das gewonnene Gelatinegranulat ist also im wesentlichen amorph, was auch röntgenometrisch oder thermoanalytisch nachweisbar ist. Diese Struktur des Granulats führt zu einer Erniedrigung des Schmelzpunktes der Gelatine und damit zu niedrigeren Verarbeitungstemperaturen, beispielsweise in einem Extruder, in welchem das Granulat anschließend zu einem Endprodukt verarbeitet wird. Aufgrund des niedrigeren Ordnungszustandes des durch Plastifizierung herkömmlicher Gelatine gewonnenen Gelatinegranulats im Vergleich mit getrockneter, handelsüblicher Gelatine, die sich durch einen hohen, teilweise kristallinen Ordnungszustand auszeichnet, ist die anschließende Formgebung auf konventionellen Thermoplastmaschinen einfacher und bei deutlich niedrigeren Temperaturen ausführbar - gleicher Wassergehalt vorausgesetzt.

Der niedrigere Schmelzpunkt des erfindungsgemäß hergestellten, amorphen Gelatinegranulats ist ein eindeutiges Unterscheidungsmerkmal gegenüber handelsüblicher Gelatine, deren Schmelzpunkt wesentlich höher liegt. Durch homogene Einmischung eines Weichmachers in das Gelatinegranulat läßt sich der Schmelzpunkt noch weiter herabsetzen.

Je nach Zusammensetzung des Gelatinegranulats lassen sich Schmelzpunkte im Bereich von 50 bis 80° C ohne weiteres realisieren.

Ein weiterer wichtiger Parameter zur Unterscheidung des erfindungsgemäßen Gelatinegranulats von herkömmlicher Gelatine (Gelatinepulver, Gelatinehydrolysat) ist der sogenannte Schmelzindex, also eine Größe, wie sie zur Kennzeichnung des Fließverhaltens von Thermoplasten verwendet wird.

Im Zusammenhang mit der Erfindung wird der Schmelzindex (MFI) nach der Vorschrift DIN 53 435 (Deutsche Norm) bestimmt: Man verwendet einen Prüfzylinder mit Kolben, füllt den Zylinder mit Gelatinegranulat und treibt bei bestimmter Temperatur und unter bestimmter Belastung des Kolbens in bestimmten Zeitabständen eine bestimmte Masse an geschmolzenem Granulat aus einer mit dem Prüfzylinder verbundenen Düse aus. Länge und Durchmesser der Düse verhalten sich wie 8 mm zu 2 mm. Auf die genannte DIN-Vorschrift wird hiermit Bezug genommen.

Der so gemessene Schmelzindex des erfindungsgemäßen Schmelzindex liegt über 1 g/10 min bei 110° C Prüftemperatur, 30 kg Gesamtbelastungsgewicht und unter Verwendung einer Standarddüse gemäß DIN A 53 735, Seite 2 mit einem Verhältnis Länge : Durchmesser von 8 mm : 2 mm.

Der Schmelzindex handelsüblicher Gelatine liegt demgegenüber praktisch bei Null, da Gelatine (oder Gelatinehydrolysat) kein thermoplastisches Fließverhalten zeigt. Konkrete Werte des Schmelzindex von erfindungsgemäßem Gelatinegranulat ergeben sich aus den nachstehenden Beispielen.

Ein weiteres eindeutiges Unterscheidungsmerkmal des erfindungsgemäßen Gelatinegranulats gegenüber herkömmlicher Gelatine oder herkömmlichem Gelatinehydrolysat ist darin zu sehen, daß das Granulat in einem elektromagnetischen Mikrowellenfeld (beispielsweise von 2450 MHz) schmilzt und/oder sich aufbläht, ein Effekt, der sich auch gewerblich nutzen läßt.

Herkömmliches Gelatinepulver oder pulverförmiges Gelatinehydrolysat zeigen diesen überraschenden Effekt nicht.

Beim Verarbeiten vorgequollener, also mit Wasser behandelter, Gelatine zu Formkörpern ist es auch schwierig, eine homogene Verteilung der Additive, insbesondere der Füllstoffe, zu erreichen, insbesondere wenn die Füllstoffe in Wasser bzw. in Weichmacher unlöslich sind. Bei der erfindungsgemäßen Herstellung des Gelatinegranulats ergibt sich hingegen eine überall gleichmäßig homogene Verteilung der Additive, insbesondere der Füllstoffe in den Gelatinekörpern des Granulats, weil hier im Verlauf der Plastifizierung des Ausgangsmaterials im Extruder eine gleichmäßige Durchmischung erfolgt und eine Trennung der Bestandteile, wie sie in einer vorgequollenen Gelatinemischung auftreten kann, bei Ausübung des erfindungsgemäßen Verfahrens unmöglich ist.

Wesentliche Vorteile des erfindungsgemäßen Gelatinegranulats bestehen in folgendem: die Herstellung erfolgt, wie beschrieben, in einfachster Weise durch Plastifizierung herkömmlicher Gelatine durch Anwendung von Wärme, Druck und Scherkraft. Dabei kann als Rohmaterial insbesondere auch eine Gelatine mit niedrigem Bloom-Wert eingesetzt werden.

Die Einarbeitung der Granulatkomponenten, insbesondere der Additive, erfolgt in die Schmelze, so daß sich eine sehr homogene Verteilung aller Zuschlagstoffe, einschließlich der ungelösten Füllstoffe, erzielen läßt. Eine Konditionierung ist bei der Verarbeitung nicht erforderlich, d. h. insbesondere, eine Quellung der Ausgangssubstanz mit Wasser oder eine Entgasung durch längeres Stehenlassen der Ausgangsmischung findet nicht statt. Die Herstellung ist im übrigen auch unabhängig von der Raumfeuchte.

Der Endverarbeiter des Granulats, also der Hersteller von Formerzeugnissen aus Gelatine, beispielsweise Kapseln, Folien, Gummibonbons, erhält ein fertiges Gelatinegranulat gleichbleibender Zusammensetzung, welches alle Zuschlagstoffe enthält und wie übliches Kunststoffgranulat ohne Vorbehandlung unbegrenzt gelagert und in einfacher Weise, beispielsweise durch direkte Eingabe in eine Spritzgußmaschine, verarbeitet werden kann. Bei der Herstellung der geformten Endprodukte sind Zusatzmaschinen, z. B. Mischer, Klimageräte, Trockner und dergleichen, nicht erforderlich.

Mit dem Gelatinegranulat kann abfallfrei gearbeitet werden, d. h. Abfälle, wie z. B. Angußstellen oder dergleichen, können direkt an der Spritzgußmaschine oder dergleichen zirkuliert werden.

Das Granulat kann einen höheren Gehalt an Zuschlagstoffen (Feststoffen mit unterschiedlicher Granulometrie und Dichte) als übliche Gelatine enthalten. Insbesondere ist eine Verarbeitung ohne zusätzliches Wasser möglich.

Beispiel 1

In die Einzugsöffnung eines Doppelschneckenextruders mit zwei gleichsinnig angetriebenen Schnecken, einem Schneckendurchmesser von 25 mm und einer Verfahrenslänge L von 48 D (48-faches des Schneckendurchmessers) wurde handelsübliche Pulvergelatine mit einer Gallertfestigkeit von 170 Bloom und einem Wassergehalt von 10,6 Gew% eindosiert. Die Dosiermenge betrug 5,0 kg/h.

An einer anderen Stelle des Extruderzylinders, die mit einem weiteren Einlaß und einem Rückschlagventil versehen war, wurde als Weichmacher Glycerin in einer Menge von 500 g/h in den Extruder gepumpt.

Das Gemisch wurde im Extruder plastifiziert, wobei die Temperatur der Zylinderheizzonen auf etwa 100° C eingestellt wurde. Die plastifizierte Masse wurde unter einem Druck von 100 bar durch eine Rundlochdüse mit sechs Bohrungen von je 2,0 mm Durchmesser extrudiert. Die abgekühlten, endlosen Stränge wurden in einem üblichen Granulator granuliert. Das erhaltene Granulat ist lagerfähig und kann auf allen konventionellen Verarbeitungsmaschinen für thermoplastischen Kunststoff, wie Spritzgießmaschinen, Extrudern, usw. verarbeitet werden.

Das Granulat bestand aus massiven Gelatinekörpern mit praktisch keinen Lufteinschlüssen (weniger als 1 Vol%), mit einer Korngröße von etwa 2 mm und mit einem Schmelzindex von 40 g/10 min.

Beispiel 2

Als Ausgangsmaterial wurde handelsübliche, pulverförmige Knochengelatine mit folgenden Qualitätsmerkmalen verwendet:

| Bloomwert | = 43 g |
| Viskosität | = 37 mP (10%ige wässrige Lösung bei 60°C) |
| Wassergehalt | = 11,9 Gew%. |

Die Gelatine wurde mit ihrer handelsüblichen Feuchte (11, 9 Gew%) ohne weitere Vorkonditionierung in die Einzugsöffnung eines gleichläufigen Zweischneckenextruders eindosiert. Die Dosiermenge betrug 7,5 kg/h. Der Extruder hatte einen Schneckendurchmesser von 25 mm und eine Verfahrenslänge von 35 D. Die Arbeitstemperatur betrug 100° C.

In eine weitere Öffnung des Extruders wurde als Plastifizierhilfsmittel (Weichmacher) Sorbitol in einer Menge von 1,875 kg/h zudosiert.

Die plastifizierte Masse wurde unter einem Druck von 20 bar durch eine Lochdüse mit vier Bohrungen von je 2,5 mm Durchmesser extrudiert, abgekühlt und mit einem Stranggranulator granuliert.

Es ergab sich ein Granulat ähnlich wie in Beispiel 1, dessen Schmelzindex jedoch unter den gleichen Bedingungen mehr als 100 g/10 min betrug.

Beispiel 3

4,4 kg/h handelsüblicher Pulvergelatine wurden in die Einzugsöffnung eines Doppelschneckenextruders mit zwei gleichsinnig angetriebenen Schnecken und einem Schneckendurchmesser von 25 mm eindosiert. Die Verfahrenslänge des Extruders betrug 48 D. Die Gelatine hatte folgende physikalische Daten:

| Bloomwert | = 161 g |
| Viskosität | = 71 mP (10%ige wässrige Lösung bei 60°C) |
| pH-Wert | = 5,5 |
| Wassergehalt | = 11,7 Gew%. |

An einer anderen Dosierstelle wurden in den Extruder 800 g/h Glycerin als Weichmacher zudosiert.

Weiterhin wurde handelsübliche Kartoffelstärke in einer Menge von 2,8 kg/h eingespeist.

Die Masse wurde bei 100° C im Extruder plastifiziert.

Nach der Extrusion durch eine Flachdüse mit einer Breite von 130 mm, welche zwischen 0,1 und 0,5 mm höhenverstellbar war, wurde mit Hilfe einer Schneidmühle granuliert.

Das erhaltene Granulat war ohne weitere Nachbehandlung lagerfähig und konnte ohne weitere Zusätze in einer üblichen Spritzgußmaschine zu Formkörpern verarbeitet werden.

Das Granulat hatte unter den oben angegebenen Prüfbedingungen einen Schmelzindex von 23 g/10 min.

Beispiel 4

In den Extruder gemäß Beispiel 1 wurde handelsübliche Pulvergelatine mit einem Bloom-Wert von etwa 100 und einem Kornspektrum von 0 bis 3,0 mm dosiert. Die Dosierrate betrug 4,5 kg/h.

Über ein Rückschlagventil wurden an einer anderen Stelle 450 g/h Glycerin eingepumpt. Die Plastifizierung erfolgte bei Temperaturen zwischen 85 und 110° C, bezogen auf mehrere Heizzonen des Extruders. In die plastifizierte Masse wurden anschließend 2,2 kg/h feinstgemahlene Kreide (Calciumcarbonat) als Füllstoff zudosiert und in der Schmelze gleichmäßig verteilt.

Anschließend wurde über Rundlochdüsen (Durchmesser 2 mm) bei einem Druck von 120 bar extrudiert und mit einem Granulator granuliert. Das erhaltene Granulat konnte ohne weitere Konditionierung auf einer Flachfolienanlage zu Tiefziehfolien verarbeitet werden.

Beispiel 5

Im Extruder gemäß Beispiel 1 wurde handelsübliche Gelatine (Bloom-Wert 50) mit einer Menge von 4,4 kg/h eingespeist. An einer weiteren Dosierstelle wurden 0,4 kg/h Glycerin in den Extruder gepumpt. Die Heizzonen des Extruders wurden auf Temperaturen zwischen 40 und 110° C aufgeheizt. Die Masse wurde bei einer Schneckendrehzahl von ca. 100 U/min und einem Druck von etwa 25 bar plastifiziert.

In die plastifizierte Masse wurden an einer dritten und vierten Dosierstelle, welche jeweils mit einem Rückschlagventil versehen waren, mit einer Pumpleistung von 100 g/h Aufschlämmungen von weißer Farbe

(TiO$_2$) bzw. roter Farbe (Eisenoxid) zudosiert.

Nach der Extrusion durch eine Düse mit vier Bohrungen von 2,5 mm Durchmesser wurde granuliert. Das erhaltene Granulat war ohne Nachtrocknung oder sonstige Konditionierung lagerfähig und thermoplastisch verarbeitbar.

Das Granulat wurde auf einer Spritzgießmaschine problemlos und auf Anhieb - bei einer Zykluszeit von 10 Sekunden - in einem Zweiplattenwerkzeug zu Formkörpern in Gestalt von Hartkapseln verarbeitet.

Der Anguß (Abfall) wurde, wie bei Kunststoff-Thermoplasten üblich, granuliert und erneut der Spritzgießmaschine zugeführt. Ein mehrmaliges Recycling des Materials war möglich, ohne daß ein Qualitätsverlust festgestellt werden konnte. Die spritzgegossenen Formkörper konnten durch alle in der Thermoplastverarbeitung üblichen Schweißmethoden, wie z. B. Heizspiegel-, Ultraschall- oder Hochfrequenzschweißen unlösbar miteinander verbunden werden.

Der Schmelzindex des Granulats betrug 60 g/10 min bei 110° C.

## Beispiel 6

Man arbeitete wie in den Beispielen 1 - 5, dosierte jedoch in den Extruder statt Gelatinepulver Gelatinehydrolysat mit einem mittleren Molekulargewicht von etwa 3000 Dalton. Man erhielt Gelatinegranulate mit den in den Beispielen 1 bis 5 angegebenen Eigenschaften, die wie ebenfalls dort angegeben, weiterverarbeitet werden konnten.

## Beispiel 7

1,0 kg/h handelsübliche Pulvergelatine mit einem Bloom-Wert von 300 werden in den Extruder gemäß Beispiel 1 eindosiert. An einer anderen Dosierstelle wurde flüssiges Eiweißhydrolysat mit einem Trockenstoffgehalt von 60 Gew% in den Extruder gepumpt, und zwar in einer Menge von 5,0 kg/h. An einer dritten Dosierstelle wurden 0,3 kg/h Glycerin zugepumpt. Die Masse wurde plastifiziert, wobei die Heizzonen des Schneckenzylinders auf Werte zwischen 40 und 135° C temperiert waren.

Durch Anlegen von Vakuum an eine der Zylinderzonen des Extruders wurde das überschüssige Wasser entfernt.

Der so erhaltene thermoplastische Werkstoff wurde über eine Rundlochdüse mit vier Bohrungen von je 2,5 mm Durchmesser extrudiert und anschließend granuliert.

Das lagerfähige, thermoplastische Gelatinegranulat hatte einen Wassergehalt von 10,0 Gew%.

Das Granulat wurde mit einer Spritzgußmaschine zu massiven Formkörpern, beispielsweise Walzen und Taschen, verarbeitet. Ein anderer Teil des Granulats wurde in einem Einschneckenextruder über eine Flachbanddüse zu klaren, schlieren- und stippenfreien Gelatinefolien verarbeitet.

## Beispiel 8

Mehrere Proben des erfindungsgemäßen Gelatinegranulats (mit und ohne Weichmacher) wurden zusammen mit mehreren Proben herkömmlicher Gelatine bzw. Gelatinehydrolysat 7 min. lang in einen handelsüblichen Mikrowellenherd (2450 MHz) gestellt. Alle Proben des Granulats blähten sich stark auf. Die Gelatine- und Gelatinehydrolysat-Proben zeigten keine Reaktion.

Dieser Effekt wurde zur Herstellung von Snacks für Diabetiker aus dem erfindungsgemäßen Gelatinegranulat ausgenutzt.

## Beispiel 9

Man verfuhr wie in Beispiel 1 und 2 beschrieben. Die Dosiermenge an Plastifiziermittel (Glycerin, Sorbitol, und dergleichen) wurde jedoch erhöht, und zwar derart, daß bei einem Gesamtmassenstrom von 5,0 kg/h an der ersten Dosierstelle 500 g/h Glycerin und an der zweiten Dosierstelle 750 g/h Sorbitol zugegeben wurden. Der Gesamtinhalt an Weichmacher bezogen auf die Gesamtmasse betrug somit 25 Gew%.

Das bei dieser Arbeitsweise erhaltene Granulat eignete sich vor allem zum Herstellen von Blasfolien sowie für die Verarbeitung zu siegelfähigen Flachfolienbändern, aus denen wiederum Gelatineweichkapseln herstellbar waren.

Das Granulat hatte unter den oben genannten Bedingungen einen Schmelzindex von 110 g/10 min.

Beispiel 10

Dieses Beispiel betrifft das Recycling von Abfällen.

Gemahlene Angüsse aus Spritzgußversuchen gemäß den voranstehenden Beispielen, sowie gemahlene Abfälle aus Spritzblas-, Flachfolien- und Granulatversuchen, welche im Durchschnitt einen Weichmachergehalt von 9% hatten, wurden in einer Menge von 5,0 kg/h in den Extruder gemäß Beispiel 1 eindosiert. Die Masse ließ sich im Extruder ohne weiteres plastifizieren.

In die plastifizierte Masse wurden mittels Dosierpumpen 100 g/h einer Aufschlämmung (1:1) von Eisenoxid und an einer weiteren Dosierstelle 1,1 kg/h einer 1%igen Aufschlämmung von Titandioxid in Glycerin zugepumpt. Der Gesamtglyceringehalt der Mischung betrug ca. 25 Gew% bezogen auf das extrudierte Granulat.

An einer anderen Dosierstelle wurde Calciumstearat in einer Menge von 1% bezogen auf die Gesamtmasse zugegeben.

Das granulierte Regenerat eignete sich sehr gut für die Herstellung von Blasfolien.

Bei der Verarbeitung des Granulats auf einem Extruder mit zwei gegenläufigen Doppelschnecken kann Luft oder ein anderes Gas, z. B. $CO_2$ so untergemischt werden, daß sich ein geschäumtes Band extrudieren läßt.

Beispiel 11

In die Einzugsöffnung des Zweischneckenextruders gemäß Beispiel 1 wurde handelsübliche Speisegelatine (Bloom-Wert ca. 60, Wassergehalt 9,5 Gew%) in einer Menge von 4,0 kg/h eingegeben. Gleichzeitig wurden 50 g/h Magnesiumstearat zudosiert. An e.-ner weiteren Dosierstelle wurden 1,0 kg/h Glycerin in den Extruder gepumpt. Die Masse wurde, wie in Beispiel 1 beschrieben, drei Minuten lang plastifiziert.

Die plastifizierte Masse wurde mit einer Massentemperatur von 120° C über eine Breitschlitzdüse (Schlitzbreite 130 mm, Schlitzhöhe 1,0 mm) extrudiert. Nach dem Abkühlen des extrudierten Bandes wurde das thermoplastische Material mit einer Schneidmühle granuliert.

Aus einem Teil dieses Granulats wurden auf einer Spritzgießmaschine Formkörper hergestellt. Aus einem anderen Teil des Granulats wurden mit einem Einschneckenextruder, an dessen Austrittsöffnung eine Flachbanddüse angebracht war, Flachfolien extrudiert, welche über einen nachgeschalteten Kalander kalibriert wurden. Aus diesen plattenförmigen Flachfolien wurden mit einer üblichen Vakuumthermoformmaschine tiefgezogene Formkörper hergestellt.

Beispiel 12

Minderwertige Gelatine in Form von Tierleim (Knochen- und Hautleim) wurde, wie im Beispiel 2 beschrieben, plastifiziert und granuliert.

Das Granulat hatte einen Wassergehalt von 8,5% und konnte in einer konventionellen Folienkalanderanlage plastifiziert und zu Bändern verarbeitet werden. Die Bänder lassen sich unmittelbar als Schmelzkleber verwenden.

Beispiel 13

Das Gelatinegranulat, welches nach einem der in den voranstehenden Beispielen beschriebenen Verfahren hergestellt worden war, wurde mit Spritzblasautomaten zu Tuben mit Gewindekopf geblasen.

Das Granulat hatte folgende Zusammensetzung:

```
Gelatine (Bloom-Wert 160)   =    65 Gew%
Sorbit                      =    25 Gew%
Wasser                      =    10 Gew%
                                100 Gew%
                                ========
```

Das Granulat wurde in einem konventionellen Spritzblasautomaten Typ Duo 30 bei 110° C plastifiziert, extrudiert und über ein Zweifachwerkzeug zu Tuben geblasen. Die Zykluszeit betrug 7 Sekunden. Die gefertigten Tuben hatten folgende Abmessungen:

| Länge | = 70 mm |
| Außendurchm. | = 15 mm |
| Wandstärke | = 1 mm |
| Gewindelänge | = 5 mm |

Beispiel 14

Bei der Herstellung von Blasfolien wurde als Ausgangsmaterial ein Gelatinegranulat, hergestellt wie in den vorangehenden Beispielen beschrieben, mit folgender Zusammensetzung verwendet:

| Gelatine mit einem Bloom-Wert von 164 | = 64 Gew% |
| Wasser | = 10 Gew% |
| Glycerin | = 25 Gew% |
| Magnesiumstearat | = 1 Gew% |
| | 100 Gew% |

Das Granulat wurde in einem Extruder zwischen 110 und 120° C plastifiziert und über eine Schlauchfoliendüse mit einem Durchmesser von 30 mm extrudiert. Der Schlauch hatte eine Wandstärke von 0,05 mm. Durch Zuführung von Blasluft wurde der Schlauchdurchmesser um ein Vielfaches erweitert und somit die Foliendicke eingestellt.

Das Material ließ sich sehr gut zu Schlauchfolien verarbeiten. Die Dehnbarkeit und die Stabilität des Extrudates war so gut, daß die Folienstärke in einem weiten Bereich variiert werden konnte.

Beispiel 15

Beispiel 14 wurde mit dem gleichen Ergebnis wiederholt, wobei jedoch das Gelatinegranulat 30 Gew% an modifizierter Maisstärke als Füllstoff enthielt.

Beispiel 16

In die Einzugsöffnung eines Doppelschneckenextruders mit zwei gleichsinnig angetriebenen Schnecken, einem Schneckendurchmesser von 25 mm und einer Verfahrenslänge L von 48 D (48-faches des Schneckendurchmessers) wurde handelsübliche Pulvergelatine mit einer Gallertfestigkeit von 84 Bloom, einer Viskosität von 4 mPa/sek. (gemessen bei 60° C in 10%iger Lsg.), einem pH-Wert von 5,5 und einem Wassergehalt von 10,2 Gew% dosiert.

Die Dosiermenge betrug 1,6 kg/h. Gleichzeitig wurde pulverförmiges Polyäthylen mit einer Menge von 6,32 kg/h in die Einzugsöffnung des Extruders dosiert.

An einer anderen Stelle des Extruders, die mit einem weiteren Einlaß und einem Rückschlagventil versehen war, wurde als Weichmacher Glycerin in einer Menge von 80g/h in den Extruder gepumpt.

Das Gemisch wurde im Extruder plastifiziert, wobei die Temperatur der Zylinderheizzonen steigend von 60 bis 115°C eingestellt wurde. Die plastifizierte Masse wurde unter einem Druck von 35 bar durch eine Rundlochdüse mit 6 Bohrungen von je 2,0 mm Durchmesser extrudiert. Die abgekühlten, endlosen Stränge wurden in einem üblichen Granulator granuliert.

Das erhaltene, lagerfähige Granulat hatte einen Wassergehalt von 2,0 Gew% und wurde auf einer konventionellen Spritzgußmaschine zu Scheiben vom 50 mm Durchmesser bei einer mittleren Stärke von 4 mm verarbeitet.

Die Heizzonen der Spritzmaschine waren auf 110 bis 130°C eingestellt. Der spezifische Einspritzdruck betrug ca. 1800 bar. Es wurde mit einer Zykluszeit von ca. 4 sek. gearbeitet.

Der Schmelzindex des Granulates war größer als 10 g/10 min.

Beispiel 17

Man verfuhr im wesentlichen wie im Beispiel 16 beschrieben.

In die Einzugsöffnung des Extruders wurde mit einer Menge von 7,5 kg/h folgende Mischung dosiert:

42,5 % Gelatine (84 Bloom, 10,2 % Wasser)
42,5 % Polyäthylen
7,5 % Calciumcarbonat
2,0 % Titandioxid
5,0 % Sorbit
0,5 % Emulgator
100,0 %

Die Heizzonen des Extruders waren auf Temperaturen zwischen 55 und 110°C eingestellt.

Es wurde ein Granulat mit einem Wassergehalt von 4,4 Gew%. erhalten.

Aus diesem Granulat wurden auf einer Spritzgießmaschine Formkörper hergestellt.

Der Schmelzindex des Granulates war größer als 10 g/10 min.

Es wurde weiterhin gefunden, daß sich außer aus Gelatine auch noch aus anderen Biopolymeren durch die beschriebenen Verfahren ein Granulat mit gleichen Eigenschaften gewinnen läßt, ein Granulat also, welches durch einfache Plastifizierung der Ausgangsstoffe hergestellt wird und für die Erzeugung von Formkörpern durch Extrudieren, Spritzen oder dergleichen geeignet ist. Herstellung und Anwendung dieser Biopolymergranulate bewegen sich dabei im Rahmen der voranstehenden Beschreibung, insbesondere der Beispiele.

Geeignete Biopolymerisate sind insbesondere Stärke, andere Proteinhydrolysate, Glutinleim, sämtliche tierische und pflanzliche Proteine sowie Hydrokolloide, beispielsweise Polysaccharide Guarmehl, Xanthan etc.

Alle im voranstehenden angegebenen, auf Gelatine bezogenen Eigenschaften und Maßnahmen lassen sich im wesentlichen ohne weiteres auf andere Biopolymerisate übertragen. Durch Anwendung der Erfindung sind somit lagerfähige, plastifizierbare Biopolymerisate in Granulatform verfügbar, die auf allen gängigen Thermoplastverarbeitungsmaschinen, z. B. Spritzguß-, Extrusions-, Tiefzieh-Druckverformmaschinen zu massiven oder hohlen Formprodukten verarbeitbar sind, wie oben im Zusammenhang mit Gelatine angegeben.

Als Ausgangsstoffe für ein Proteingranulat eignen sich insbesondere auch Frischknochen, entfettetes Knochenschrot, Ossein, Schweineschwarte, zerkleinerte Rinder- bzw. Kalbshaut. Der Ausgangsstoff wird mit den üblichen Additiven, gegebenenfalls zusammen mit einem Enzym, in den Extruder eingebracht und unter Anwendung von Druck, erhöhter Temperatur und Scherkraft plastifiziert. Die plastifizierte Masse wird extrudiert und das Extrudat granuliert.

**Patentansprüche**

1.  Gelatinegranulat, enthaltend

    massive Gelatinekörper mit einem Wassergehalt von 1 - 12 Gew%, mit einer Korngröße von 0,1 bis 10 mm, mit einem Gehalt an Lufteinschlüssen von weniger als 1 Vol% und mit einem Schmelzindex (MFI) größer als 1 g/10 min bei 110°C Prüftemperatur, 30 kg Gesamtbelastungsgewicht und unter Verwendung einer Standarddüse gemäß DIN A 53 735, Seite 2 mit einem Verhältnis Länge : Durchmesser von 8 mm : 2 mm.

2.  Gelatinegranulat nach Anspruch 1, gekennzeichnet durch einen Wassergehalt von 8 - 12 Gew%.

3.  Gelatinegranulat nach Anspruch 1, gekennzeichnet durch einen Gehalt an Additiven von 0,1 - 80 Gew%.

4.  Gelatinegranulat nach Anspruch 3, gekennzeichnet durch einen Gehalt an Weichmacher von 1 - 50 Gew%.

5.  Gelatinegranulat nach Anspruch 4, gekennzeichnet durch einen Gehalt an Weichmacher von 5 - 30 Gew%.

6.  Gelatinegranulat nach Anspruch 4, gekennzeichnet durch einen Gehalt an Weichmacher von 5 - 10 Gew%.

**7.** Gelatinegranulat nach Anspruch 4, dadurch gekennzeichnet, daß der Weichmacher Glycerin, Mannit, Sorbit, ein Fett, eine Fettsäure, eine Seife, eine modifizierte Stärke, native Stärke, Äthylenglykol, Polyäthylenglykol, Monoglycerinacetat, Diglycerinacetat, Triglycerinacetat, Zuckerfettsäureester, Dimethylensulfoxid, 2,2,2 Trifluoräthanol und/oder Propylenglykol ist.

**8.** Gelatinegranulat nach Anspruch 3, gekennzeichnet durch einen Gehalt an Vernetzungsmittel von 0,1 - 10 Gew%.

**9.** Gelatinegranulat nach Anspruch 8, gekennzeichnet durch einen Gehalt an Vernetzungsmittel von 0,5 - 5 Gew%.

**10.** Gelatinegranulat nach Anspruch 8, dadurch gekennzeichnet, daß der Härter ein Aldehyd, Dialdehyd, ein Molekül mit mehrfacher Aldehydfunktion, ein Diisocyanat, eine Dialdehydstärke, Acrylsäure, Acrylat, Hexamethylendiamin, Styrol reaktives Melaminderivat und/oder Acrolein ist.

**11.** Gelatinegranulat nach Anspruch 3, gekennzeichnet durch einen Gehalt an Füllstoffen von 1 - 80 Gew%.

**12.** Gelatinegranulat nach Anspruch 11 , gekennzeichnet durch einen Gehalt an Füllstoffen von 20 - 60 Gew%.

**13.** Gelatinegranulat nach Anspruch 12, gekennzeichnet durch einen Gehalt an Füllstoffen von 30 - 50 Gew%.

**14.** Gelatinegranulat nach Anspruch 11, dadurch gekennzeichnet, daß der Füllstoff Calciumcarbonat, Holzmehl, Baumwolle, Kohlehydrat (Stärke und/oder Zucker), Dicalciumphosphat, Fleischmehl, Knochenmehl, Fischmehl, Milcheiweiß, Sojamehl, Fett, Fettsäure, Polyvinylalkohol, Talkum, Polyäthylen, Polypropylen, Polyamid und/oder Polyester ist.

**15.** Gelatinegranulat nach Anspruch 1, gekennzeichnet durch einen Gehalt an Farbstoff zwischen 0,001 und 5 Gew%.

**16.** Gelatinegranulat nach Anspruch 15, gekennzeichnet durch einen Gehalt an Farbstoff zwischen 0,1 und 3 Gew%.

**17.** Gelatinegranulat nach Anspruch 15, gekennzeichnet durch einen Gehalt an Farbstoff zwischen 0,5 und 2 Gew%.

**18.** Verfahren zur Herstellung eines Gelatinegranulats nach einem der Ansprüche 1 bis 17, dadurch gekennzeichnet, daß man als Ausgangsstoff Pulvergelatine und/oder gepulvertes Gelatinehydrolysat, gegebenenfalls zusammen mit einem Weichmacher, einem Härter, einem Füllstoff und/oder einem Farbstoff, in einen Extruder einbringt, den Ausgangsstoff im Extruder unter Anwendung von Druck, Scherkraft und erhöhter Temperatur plastifiziert, die plastifizierte Masse extrudiert und das Extrudat granuliert.

**19.** Verfahren nach Anspruch 18, dadurch gekennzeichnet, daß man bei einem Druck zwischen 1 und 300 bar plastifiziert.

**20.** Verfahren nach Anspruch 18, dadurch gekennzeichnet, daß man bei einem Druck zwischen 3 und 100 bar plastifiziert.

**21.** Verfahren nach Anspruch 18, dadurch gekennzeichnet, daß man bei einem Druck zwischen 5 und 50 bar plastifiziert.

**22.** Verfahren nach Anspruch 18, dadurch gekennzeichnet, daß man bei einer Temperatur zwischen 30 und 250° C plastifiziert.

**23.** Verfahren nach Anspruch 18, dadurch gekennzeichnet, daß man bei einer Temperatur zwischen 50 und 150° C plastifiziert.

**24.** Verfahren nach Anspruch 18, dadurch gekennzeichnet, daß man bei einer Temperatur zwischen 70 und 130° C plastifiziert.

**25.** Verfahren nach Anspruch 18, dadurch gekennzeichnet, daß man während einer Zeitdauer von 5 sec - 30 min plastifiziert.

**26.** Verfahren nach Anspruch 18, dadurch gekennzeichnet, daß man während einer Zeitdauer von 0,5 min - 10 min plastifiziert.

**27.** Verfahren nach Anspruch 18, dadurch gekennzeichnet, daß man während einer Zeitdauer von 1 bis 5 min plastifiziert.

**28.** Verfahren nach Anspruch 18, dadurch gekennzeichnet, daß man Pulvergelatine und/oder gepulvertes Gelatinehydrolysat sowie die Zuschlagstoffe an separaten Dosierstellen direkt in den Extruder eingibt.

**29.** Verfahren nach Anspruch 18, dadurch gekennzeichnet, daß man einen endlos extrudierten Strang im Luftstrom abkühlt und den Strang mit Schneidmühlen granuliert.

**30.** Vorrichtung zur Durchführung des Verfahrens nach einem der Ansprüche 18 bis 29, gekennzeichnet durch einen Doppelschneckenextruder mit einem Durchmesser/Längen (D/L)-Verhältnis von mindestens 35.

**31.** Vorrichtung nach Anspruch 30, gekennzeichnet durch einen Doppelschneckenextruder mit zwei gleichsinnig angetriebenen Schnecken.

**Claims**

**1.** A gelatin granulate, containing solid gelatin bodies having a water content of 1 - 12 wt.%, a grain size of 0.1 to 10mm, an air inclusion content of less than 1 vol.% and a melt flow index (M.F.I.) of greater than 1g / 10 min. at a testing temperature of 110° C, 30kg total load weight and using a standard nozzle according to DIN A 53 735, page 2 with a length : diameter ratio of 8mm : 2mm.

**2.** A gelatin granulate according to claim 1, characterised by a water content of 8 - 12 wt.%.

**3.** A gelatin granulate according to claim 1, characterised by an additives content of 0.1 - 80 wt.%.

**4.** A gelatin granulate according to claim 3, characterised by a plasticiser content of 1 - 50 wt.%.

**5.** A gelatin granulate according to claim 4, characterised by a plasticiser content of 5 - 30 wt.%.

**6.** A gelatin granulate according to claim 4, characterised by a plasticiser content of 5 - 10 wt.%.

**7.** A gelatin granulate according to claim 4, characterised in that the plasticiser is glycerin, mannitol, sorbitol, a fat, a fatty acid, a soap, a modified starch, native starch, ethylene glycol, polyethylene glycol, monoglycerin acetate, diglycerin acetate, triglycerin acetate, sugar fatty acid ester, dimethyl sulphoxide, 2,2,2-trifluoroethanol and/or propylene glycol.

**8.** A gelatin granulate according to claim 3, characterised by a cross-linking agent content of 0.1 - 10 wt.%.

**9.** A gelatin granulate according to claim 8, characterised by a cross-linking agent content of 0.5 - 5 wt.%.

**10.** A gelatin granulate according to claim 8, characterised in that the hardener is an aldehyde, dialdehyde, a molecule having a multiple aldehyde function, a diisocyanate, a dialdehyde starch, acrylic acid, acrylate, hexamethylene diamine, styrene, reactive melamine derivative and/or acrolein.

**11.** A gelatin granulate according to claim 3, characterised by a filler content of 1 - 80 wt.%.

**12.** A gelatin granulate according to claim 11, characterised by a filler content of 20 - 60 wt.%.

**13.** A gelatin granulate according to claim 12, characterised by a filler content of 30 - 50 wt.%.

**14.** A gelatin granulate according to claim 11, characterised in that the filler is calcium carbonate, wood flour, cotton, carbohydrate (starch and/or sugar), dicalcium phosphate, meat meal, bone meal, fish meal, milk protein, soya meal, fat, fatty acid, polyvinyl alcohol, talcum, polyethylene, polypropylene, polyamide and/or polyester.

**15.** A gelatin granulate according to claim 1, characterised by a dye content between 0.001 and 5 wt.%.

**16.** A gelatin granulate according to claim 15, characterised by a dye content between 0.1 and 3 wt.%.

**17.** A gelatin granulate according to claim 15, characterised by a dye content between 0.5 and 2 wt.%.

**18.** A method of preparing a gelatin granulate according to any one of claims 1 to 17, characterised in that powdered gelatin and/or powdered gelatin hydrolysate, if appropriate together with a plasticiser, a hardener, a filler and/or a dye, is introduced, as starting material, into an extruder, the starting material is plasticised in the extruder under the application of pressure, shearing force and increased temperature, the plasticised mass is extruded and the extrudate is granulated.

**19.** A method according to claim 18, characterised in that the plasticising process takes place at a pressure between 1 and 300 bar.

**20.** A method according to claim 18, characterised in that the plasticising process takes place at a pressure between 3 and 100 bar.

**21.** A method according to claim 18, characterised in that the plasticising process takes place at a pressure between 5 and 50 bar.

**22.** A method according to claim 18, characterised in that the plasticising process takes place at a temperature between 30 and 250°C.

**23.** A method according to claim 18, characterised in that the plasticising process takes place at a temperature between 50 and 150°C.

**24.** A method according to claim 18, characterised in that the plasticising process takes place at a temperature between 70 and 130°C.

**25.** A method according to claim 18, characterised in that the plasticising process takes place during a period of 5 sec. - 30 min.

**26.** A method according to claim 18, characterised in that the plasticising process takes place during a period of 0.5 min. - 10 min.

**27.** A method according to claim 18, characterised in that the plasticising process takes place during a period of 1 to 5 min.

**28.** A method according to claim 18, characterised in that powdered gelatin and/or powdered gelatin hydrolysate and the loading materials are introduced directly into the extruder at separate dosing points.

**29.** A method according to claim 18, characterised in that an endlessly extruded strand is cooled in an air flow and the strand is granulated by means of cutting mills.

**30.** A device for carrying out the method according to any one of claims 18 to 29, characterised by a twin-screw extruder with a diameter/length (D/L) ratio of at least 35.

EP 0 354 345 B1

**31.** A device according to claim 30, characterised by a twin-screw extruder having two screws driven in the same direction.

**Revendications**

**1.** Granulat de gélatine, contenant des corps solides de gélatine avec une teneur en eau de 1 à 12 % en poids, avec une taille de grains de 0,1 à 10 mm, avec une teneur en inclusions d'air inférieure à 1 % en volume, et avec un indice de fusion (MFI) supérieur à 1 g/10 min. à une température d'essai de 110° C, un poids total de contrainte de 30 kg et, en utilisant une fillère d'extrusion standard selon la norme DIN A 53735, page 2, présentant un rapport de la longueur au diamètre de 8mm : 2mm.

**2.** Granulat de gélatine selon la revendication 1, caractérisé par une teneur en eau de 8 à 12 % en poids.

**3.** Granulat de gélatine selon la revendication 1, caractérisé par une teneur en additifs de 0,1 à 80 % en poids.

**4.** Granulat de gélatine selon la revendication 3, caractérisé par une teneur en plastifiant de 1 à 50 % en poids.

**5.** Granulat de gélatine selon la revendication 4, caractérisé par une teneur en plastifiant de 5 à 30 % en poids.

**6.** Granulat de gélatine selon la revendication 4, caractérisé par une teneur en plastifiant de 5 à 10 % en poids.

**7.** Granulat de gélatine selon la revendication 4, caractérisé en ce que le plastifiant est de la glycérine, du mannitol, du sorbitol, une graisse, un acide gras, un savon, un amidon modifié, un amidon natif, de l'éthylène glycol, du polyéthylène glycol, du monoacétate de glycérine, du diacétate de glycérine, du triacétate de glycérine, un ester d'acide gras de sucre, du sulfoxyde de diméthylène, du 2,2,2-trifluoréthanol et/ou du propylène glycol.

**8.** Granulat de gélatine selon la revendication 3, caractérisé par une teneur en réticulant de 0,1 à 10 % en poids.

**9.** Granulat de gélatine selon la revendication 8, caractérisé par une teneur en réticulant de 0,5 à 5 % en poids.

**10.** Granulat de gélatine selon la revendication 8, caractérisé en ce que le durcisseur est un aldéhyde, un dialdéhyde, une molécule à fonction aldéhyde multiple, un diisocyanate, un amidon dialdéhyde, de l'acide acrylique, de l'acrylate, de l'hexaméthylène-diamine, un dérivé de mélamine réagissant au styrène et/ou de l'acroléine.

**11.** Granulat de gélatine selon la revendication 3, caractérisé par une teneur en charges de 1 à 80 % en poids.

**12.** Granulat de gélatine selon la revendication 11, caractérisé par une teneur en charges de 20 à 60 % en poids.

**13.** Granulat de gélatine selon la revendication 12, caractérisé par une teneur en charges de 30 à 50 % en poids.

**14.** Granulat de gélatine selon la revendication 11, caractérisé en ce que la charge est du carbonate de calcium, de la sciure de bois, du coton, un glucide (amidon et/ou sucre), du diphosphate de calcium, de la farine de viande, de la farine d'os, de la farine de poisson, de la lactoprotéine, de la farine de soja, de la graisse, un acide gras, de l'alcool polyvinylique, du talc, du polyéthylène, du polypropylène, un polyamide et/ou un polyester.

13

**15.** Granulat de gélatine selon la revendication 1, caractérisé par une teneur en colorant comprise entre 0,001 et 5 % en poids.

**16.** Granulat de gélatine selon la revendication 15, caractérisé par une teneur en colorant comprise entre 0,1 et 3 % en poids.

**17.** Granulat de gélatine selon la revendication 15, caractérisé par une teneur en colorant comprise entre 0,5 et 2 % en poids.

**18.** Procédé de fabrication d'un granulat de gélatine selon l'une quelconque des revendications 1 à 17, caractérisé en ce qu'on introduit comme matière première dans une boudineuse de la gélatine en poudre et/ou de l'hydrolysat de gélatine pulvérisé, éventuellement conjointement avec un plastifiant, un durcisseur, une charge et/ou un colorant, on plastifie la matière première dans la boudineuse en utilisant une pression, une force de cisaillement et une température accrue, on extrude la masse plastifiée et on granule le produit d'extrusion.

**19.** Procédé selon la revendication 18, caractérisé en ce qu'on plastifie à une pression comprise entre 1 et 300 bars.

**20.** Procédé selon la revendication 18, caractérisé en ce qu'on plastifie à une pression comprise entre 3 et 100 bars.

**21.** Procédé selon la revendication 18, caractérisé en ce qu'on plastifie à une pression comprise entre 5 et 50 bars.

**22.** Procédé selon la revendication 18, caractérisé en ce qu'on plastifie à une température comprise entre 30 et 250° C.

**23.** Procédé selon la revendication 18, caractérisé en ce qu'on plastifie à une température comprise entre 50 et 150° C.

**24.** Procédé selon la revendication 18, caractérisé en ce qu'on plastifie à une température comprise entre 70 et 130° C.

**25.** Procédé selon la revendication 18, caractérisé en ce qu'on plastifie pendant une durée de 5 secondes à 30 minutes.

**26.** Procédé selon la revendication 18, caractérisé en ce qu'on plastifie pendant une durée de 0,5 minute à 10 minutes.

**27.** Procédé selon la revendication 18, caractérisé en ce qu'on plastifie pendant une durée de 1 à 5 minutes.

**28.** Procédé selon la revendication 18, caractérisé en ce qu'on introduit directement dans la boudineuse, en des endroits de dosage séparés, de la gélatine en poudre et/ou de l'hydrolysat de gélatine pulvérisé ainsi que les additifs.

**29.** Procédé selon la revendication 18, caractérisé en ce qu'on refroidit par courant d'air un boudin extrudé sans fin et on granule le boudin au moyen de broyeurs coupants.

**30.** Dispositif pour la mise en oeuvre du procédé selon l'une quelconque des revendications 18 à 29, caractérisé par une boudineuse à deux vis avec un rapport du diamètre à la longueur (D/L) d'au moins 35.

**31.** Dispositif selon la revendication 30, caractérisé par une boudineuse à deux vis avec deux vis entraînées dans le même sens.